# EUROPÄISCHE PATENTSCHRIFT

(11) **EP 0 968 685 B1**
(45) Veröffentlichungstag und Bekanntmachung des Hinweises auf die Patenterteilung: **01.09.2004**
(21) Anmeldenummer: 99112136.9
(22) Anmeldetag: 24.06.1999
(51) Int. Cl.: A61B 17/74

(54) **Vorrichtung zur Versorgung von Knochenbrüchen**
Device for treating bone fractures
Dispositif pour le traitement des fractures des os

(30) Priorität: 30.06.1998 DE 19829228
(43) Veröffentlichungstag der Anmeldung: 05.01.2000
(73) Patentinhaber: Aesculap AG & Co. KG, 78532 Tuttlingen (DE)
(72) Erfinder: Böttiger, Roland, 78604 Rietheim-Weilheim (DE); Stedtfeld, Hans-Werner, 90475 Nürnberg (DE); Saueressig, Thomas, 78532 Tuttlingen (DE)
(74) Vertreter: Böhme, Ulrich, Dr. Dipl.-Phys.

(56) Entgegenhaltungen:
- EP-A- 0 599 752
- EP-A- 0 640 318
- EP-A- 0 727 189
- DE-U- 29 709 725

## Beschreibung

Die Erfindung betrifft eine Vorrichtung zur Versorgung von Knochenbrüchen gemäß dem Oberbegriff von Anspruch 1. Der nächstliegende Stand der Technik ist DE 29 709 725 U.

Derartige Vorrichtungen sind bekannt beispielsweise aus der EP 0 727 189 B1 oder der EP 0 599 752 B1.

Bei derartigen Vorrichtungen ist es notwendig, zur Stabilisierung der Knochenfragmente die Schenkelhalsschraube in der Hülse sicher zu führen, und zu diesem Zweck muß die Hülse im Verriegelungsnagel sicher festgelegt werden. Diese Hülse wird bei den bekannten Vorrichtungen erst über die Schenkelhalsschraube geschoben, wenn diese bereits in dem zu fixierenden Knochenfragment festgelegt ist, und daher müssen spezielle Vorrichtungen vorgesehen werden, um die Hülse nach dem Aufschieben auf die Schenkelhalsschraube im Verriegelungsnagel festzulegen.

Bei der Vorrichtung gemäß EP 0 727 189 B1 erfolgt diese Festlegung durch eine in Längsrichtung in den Verriegelungsnagel einschraubbare Feststellschraube, die im Bereich von Umfangsrippen gegen die Außenwand der Hülse drückt. Dazu ist es notwendig, in den Verriegelungsnagel von oben her die Feststellschraube einzuschrauben, und dies erfordert einen erhöhten konstruktiven Aufwand, außerdem wird dadurch der Verriegelungsnagel gegebenenfalls geschwächt.

Bei der Konstruktion gemäß EP 0 599 752 B1 wird die Hülse in die Durchgangsbohrung eingeschraubt, die zu diesem Zweck über ihre gesamte Länge mit einem Innengewinde versehen werden muß. Außerdem muß bei der bekannten Vorrichtung ein Anschlagbund an der Hülse vorgesehen werden, um deren Einschraubtiefe zu begrenzen.

Es ist Aufgabe der Erfindung, eine gattungsgemäße Vorrichtung so auszugestalten, daß ohne Schwächung des Verriegelungsnagels und mit möglichst wenig konstruktiven Anpassungen des Verriegelungsnagels und der Hülse eine sichere Festlegung der Hülse im Verriegelungsnagel erreichbar ist.

Diese Aufgabe wird bei einer Vorrichtung der eingangs beschriebenen Art erfindungsgemäß dadurch gelöst, daß die Hülse an einem Ende ein über ihren Außenmatel überstehendes Außengewinde trägt und daß der Durchgangsbohrung in dem Verriegelungsnagel eine stufenförmige Erweiterung vorgelagert ist, die an ihrer Seitenwand mindestens eine im wesentlichen in Umfangsrichtung verlaufende Rippe aufweist, die beim Einschrauben der Hülse in die Durchgangsbohrung zwischen die Gewindegänge des Außengewindes der Hülse eingreift.

Bei dieser Konstruktion bleibt also die Durchgangsbohrung über ihre gesamte Länge glatt, und ebenso bleibt der Außenumfang der Hülse im Einsteckbereich in die Durchgangsbohrung glatt, so daß beide Teile mit geringem Spiel exakt relativ zueinander geführt werden können. Der Verriegelungsnagel erfährt dadurch keine Schwächung, eine glatt durchgehende zylindrische Bohrung ist einfach herzustellen. Die Festlegung der Hülse erfolgt dadurch, daß das Außengewinde der Hülse und die Rippe oder die Rippen auf der Seitenwand der Erweiterung ineinander eingreifen. Beim Einschrauben der Hülse in die Durchgangsbohrung kann die Hülse dadurch so lange eingeschraubt werden, bis das Außengewinde auf der Hülse gegen die Stufe der stufenförmigen Erweiterung anschlägt. Damit erhält man eine definierte Lage der Hülse im Verriegelungsnagel, in der die Hülse im Verriegelungsnagel durch den Eingriff der Rippe oder der Rippen in das Außengewinde sicher festgelegt ist.

Die Rippe kann durch einen Gewindegang eines Innengewindes der Seitenwand gebildet sein, so daß diese beiden Gewinde an der Seitenwand einerseits und auf der Außenseite der Hülse andererseits beim Einschrauben der Hülse ineinander greifen.

Es kann aber bei einer anderen Ausführungsform auch vorgesehen sein, daß die Rippe exakt in einer Ebene verläuft, die senkrecht auf der Längsachse der Durchgangsbohrung steht. In diesem Falle ist die Rippe nicht Teil eines Gewindeganges, weist also keine Steigung auf, trotzdem kann eine solche Rippe bei entsprechender Bemessung zwischen die Gewindegänge des Außengewindes der Hülse eingreifen und die Hülse dadurch in axialer Richtung festlegen.

Günstig ist es, wenn die Seitenwand der Erweiterung mehrere parallel zueinander verlaufende und nebeneinander liegende Rippen trägt, deren Abstand voneinander gleich ist. Es kann sich dabei sowohl um die Gewindegänge eines Gewindes handeln als auch um parallel zueinander verlaufende Rippen, die keine Steigung in Längsrichtung aufweisen.

Grundsätzlich wäre es möglich, daß sich die Erweiterung über den gesamten Umfang der Durchgangsbohrung erstreckt, bei einer bevorzugten Ausführungsform ist jedoch vorgesehen, daß sich die Erweiterung nur über einen Teil des Umfanges der Durchgangsbohrung erstreckt.

Insbesondere kann vorgesehen sein, daß die Erweiterung bei einer schräg durch den Verriegelungsnagel führenden Durchgangsbohrung an der Seite der Durchgangsbohrung anschließt, die am weitesten über eine senkrecht auf der Längsachse der Durchgangsbohrung stehenden Ebene hervorsteht. Bei einer schräg durch den Verriegelungsnagel führenden Durchgangsbohrung ist die Austrittsebene der Durchgangsbohrung im Winkel angeordnet zu einer Ebene, die senkrecht auf der Längsachse der Durchgangsbohrung liegt. Dadurch wird die Durchgangsbohrung auf einer Seite länger als auf der gegenüberliegenden Seite, und an dieser längeren Seite schließt sich bei dieser Ausgestaltung die stufenförmige Erweiterung an, die somit weiterhin innerhalb des Außenumfangs des Verriegelungsnagels bleibt.

Bei einer weiteren bevorzugten Ausführungsform der Erfindung ist vorgesehen, daß parallel zur Durchgangsbohrung eine zweite Durchgangsbohrung in dem Verriegelungsnagel angeordnet ist, welche einen Antirotationsstift aufnimmt. Dieser parallel zu der Verriegelungsschraube verlaufende Antirotationsstift dringt ebenfalls in das zu fixierende Knochenfragment ein und sichert dieses somit gegen eine Drehung um die Längsachse der Schenkelhalsschraube.

Günstig ist es, wenn der Antirotationsstift in die zweite Durchgangsbohrung eingeschraubt ist, dadurch läßt er sich in dieser festlegen. Das Gewinde kann dabei so ausgebildet sein, daß es sich nur über einen Teil der zweiten Durchgangsöffnung erstreckt, so daß die Einschraubtiefe dadurch begrenzt wird, daß das Ende des Außengewindes des Antirotationsstiftes gegen das Ende des Innengewindes in der zweiten Durchgangsbohrung anschlägt.

Vorzugsweise weist der Antirotationsstift über seine aus dem Verriegelungsnagel hervorstehende Länge eine glatte zylindrische Außenwand auf, so daß eine Axialverschiebung zwischen Knochenfragment einerseits und Antirotationsstift andererseits nicht behindert wird.

Dies kann noch dadurch unterstützt werden, daß der Antirotationsstift ein spitzes Ende aufweist, sich also gegebenenfalls bei einer Annäherung der Knochenfragmente im Verlauf des Heilungsprozesses auch tiefer in das Knochenfragment eingraben kann.

Dieser Vorgang kann weiterhin dadurch unterstützt werden, daß der Antirotationsstift einen Außendurchmesser aufweist, der wesentlich kleiner ist als der der Hülse, beispielsweise kann dieser kleiner sein als der halbe Durchmesser der Hülse.

Die nachfolgende Beschreibung bevorzugter Ausführungsformen der Erfindung dient im Zusammenhang mit der Zeichnung der näheren Erläuterung. Es zeigen:

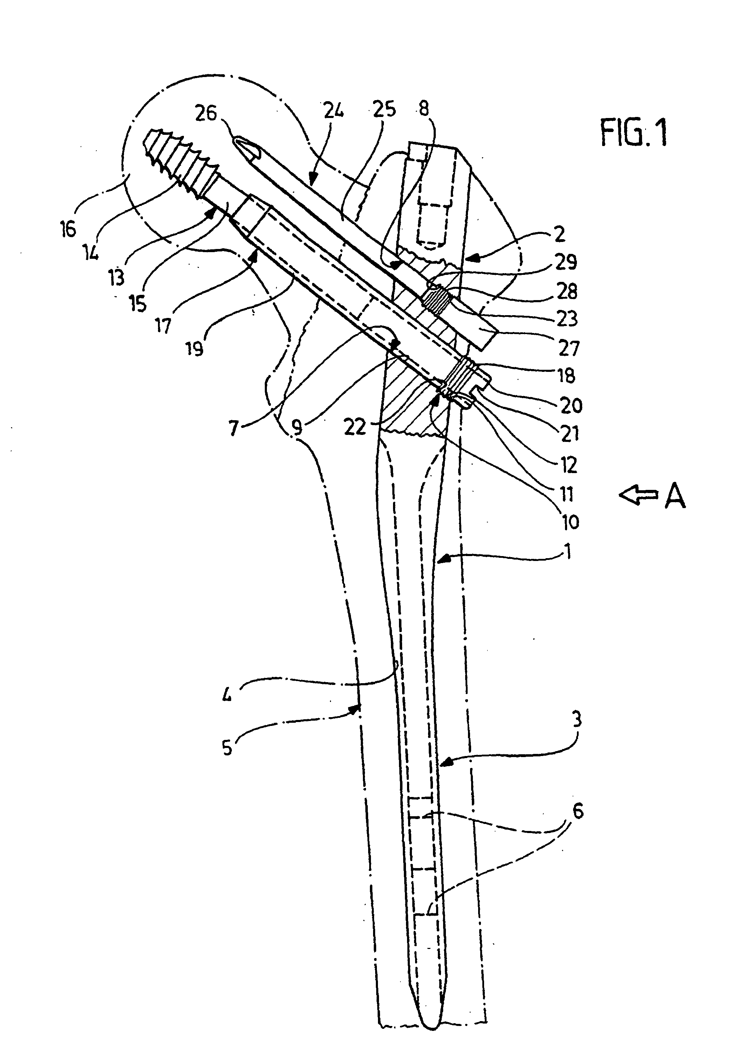

- Figur 1:: eine Längsschnittansicht einer in den Femur eingesetzten Verriegelungsvorrichtung und

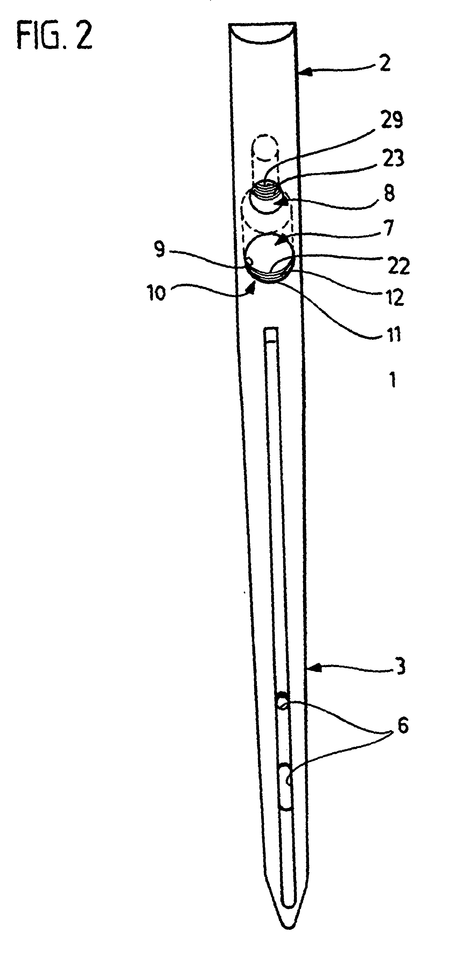

- Figur 2:: eine Ansicht des Verriegelungsnagels der Vorrichtung der Figur 1 in Richtung des Pfeiles A in Figur 1.

Die in der Zeichnung dargestellte Vorrichtung zur Versorgung eines Oberschenkelhalsbruches umfaßt einen Verriegelungsnagel 1 mit einem zylindrischen Oberteil 2 und einem sich daran anschließenden, verjüngten Schaft 3. Dieser Verriegelungsnagel 1 wird in den Markraum 4 eines Femurknochens 5 eingesetzt und dort durch quer in den Femurknochen 5 eingedrehte, in der Zeichnung nicht dargestellte Schrauben festgelegt, die durch Querbohrungen 6 im Bereich des Schaftes 3 hindurchtreten. Im eingesetzten Zustand wird der Verriegelungsnagel 1 dadurch praktisch vollständig im Markraum 4 aufgenommen.

Im Schaft 3 sind nebeneinander zwei parallel verlaufende Durchgangsbohrungen 7 und 8 angeordnet, die den Schaft 3 quer durchsetzen und gegenüber der Längsachse des Schaftes 3 schräg geneigt sind, beispielsweise um einen Winkel von 50°.

Die erste Durchgangsbohrung 7, die näher am Schaft 3 liegt, weist eine glatte, kreiszylindrische Innenwand 9 auf, deren Durchmesser über die gesamte Länge der Durchgangsbohrung 7 gleich ist. Lediglich an einem Ende der Durchgangsbohrung 7 vergrößert sich der Innendurchmesser der Durchgangsbohrung 7 stufenförmig und bildet somit eine Erweiterung 10 aus, deren zylindrische Innenwand 11 mehrere parallel zueinander verlaufende Rippen 12 trägt. Diese können durch die Schraubgänge eines Innengewindes auf der Innenwand 11 ausgebildet sein, die Rippen können aber auch genau in Umfangsrichtung verlaufen, also ohne Steigung in Längsrichtung.

Die Tiefe der Erweiterung 10 ist so gering, daß die Erweiterung 10 aufgrund des schrägen Verlaufs der Durchgangsbohrung 7 im Schaft 3 sich nicht über den gesamten Umfangsbereich der Durchgangsbohrung 7 erstreckt, sondern nur über den unteren Teil, also den Teil, der in den Umriß des Schaftes 3 eintaucht, auf der gegenüberliegenden Seite endet dagegen die Durchgangsbohrung 7 mit der glatten, zylindrischen Innenwand 9.

Die Erweiterung 10 bildet also bei dem dargestellten Ausführungsbeispiel eine muldenförmige Vertiefung am unteren Ende der ansonsten vollständig zylindrischen Durchgangsbohrung 7 aus, und in dieser muldenförmigen Vertiefung sind die Rippen 12 angeordnet.

Durch die Durchgangsbohrung 7 wird eine Verriegelungsschraube 13 hindurchgeschoben, die an ihrem kopfseitigen Ende ein gegebenenfalls selbstschneidendes Knochengewinde 14 trägt, an welches sich ein zylindrischer Schaft 15 anschließt, dessen Außendurchmesser deutlich kleiner ist als der Innendurchmesser der Innenwand 9 der Durchgangsbohrung 7. Der Außendurchmesser des Knochengewindes 14 ist so gewählt, daß die Verriegelungsschraube 13 durch die Durchgangsbohrung 7 hindurchgeschoben und mittels eines in der Zeichnung nicht dargestellten geeigneten Werkzeuges in das zu fixierende Knochenfragment 16 eingeschraubt werden kann.

Über den zylindrischen Schaft 15 der in dieser Weise eingeschraubten Verriegelungsschraube 13 wird eine Hülse 17 geschoben, deren Innendurchmesser dem Außendurchmesser des Schaftes 15 entspricht und deren Außendurchmesser dem Innendurchmesser der Durchgangsbohrung 7 entspricht. Die Hülse 17 füllt somit den Zwischenraum zwischen dem Schaft 15 der Verriegelungsschraube 13 und der Innenwand 9 der Durchgangsbohrung 7 aus.

Diese Hülse 17 trägt an ihrem dem Knochengewinde 14 abgewandten Ende ein Außengewinde 18, dessen Schraubgänge nach außen über den zylindrischen Außenmantel 19 der Hülse 17 hervorstehen. An der dem Außengewinde 18 benachbarten Stirnkante 20 sind Vertiefungen 21 angeordnet, die ein in der Zeichnung nicht dargestelltes Drehwerkzeug aufnehmen können, mit dessen Hilfe die Hülse 17 in der Durchgangsbohrung 7 verdreht werden kann. Bei diesem Verdrehen greifen das Außengewinde 18 auf der Hülse 17 und die Rippen 12 in der Erweiterung 10 ineinander, bis das vordere Ende des Außengewindes 18 an der Stufe 22 anschlägt, die zwischen der Innenwand 9 der Durchgangsbohrung 7 einerseits und der Innenwand 11 der Erweiterung 10 andererseits ausgebildet ist. Dadurch wird die Einschraubtiefe der Hülse 17 begrenzt.

Die Hülse 17 liegt bei dieser Konstruktion somit praktisch über ihre gesamte Länge mit dem zylindrischen Außenmantel 19 an der ebenfalls zylindrischen Innenwand 9 der Durchgangsbohrung 7 an und erfährt dort über ihre gesamte Länge eine optimale Führung, lediglich im Bereich der in Längsrichtung der Durchgangsbohrung sehr kurzen Erweiterung 10 ergibt sich ein Eingriff mit dem Außengewinde 18, und damit ist der Schaft 3 des Verriegelungsnagels 1 im Austrittsbereich der Durchgangsbohrung 7 nur geringfügig gegenüber einer Konstruktion modifiziert, die keine derartige Erweiterung mit entsprechenden Rippen aufweist. Die Hülse 17 ist ebenfalls sehr einfach ausgebildet, ihr Außenmantel ist über die gesamte Länge gleichbleibend zylindrisch ausgebildet, lediglich in einem kurzen Bereich ist auf diesen Außenmantel das Außengewinde 18 aufgesetzt.

Die zweite Durchgangsbohrung 8 weist einen wesentlich kleineren Durchmesser auf als die erste Durchgangsbohrung 7, beispielsweise kann der Innendurchmesser der Durchgangsbohrung 8 kleiner als 50% des Innendurchmessers der ersten Durchgangsbohrung 7 sein.

Die Durchgangsbohrung 8 ist dabei von der Einschubseite her sich stufig verengend ausgebildet, im erweiterten Teil trägt sie ein Innengewinde 23, sonst sind die Innenwände glatt ausgeführt.

In diese zweite Durchgangsbohrung 8 ist ein Antirotationsstift 24 eingeschraubt, der einen glatten, zylindrischen Schaft 25 mit einer Spitze 26 aufweist und einen sich daran anschließenden, verdickten zylindrischen Abschnitt 27, der im Übergangsbereich zu dem Schaft 25 ein Außengewinde 28 trägt. Der Außendurchmesser des Schaftes 25 entspricht dem Innendurchmesser der Durchgangsbohrung 8 im Abschnitt mit geringerem Innendurchmesser, der Außendurchmesser des verdickten Abschnittes 27 des Antirotationsstiftes 24 dagegen dem Außendurchmesser der zweiten Durchgangsbohrung 8 im Bereich mit größerem Innendurchmesser. Beim Einschrauben des Antirotationsstiftes 24 in die Durchgangsbohrung 8 stößt das Außengewinde 28 gegen die Stufe 29 in der Durchgangsbohrung 8 und begrenzt somit die Einschraubtiefe.

Der Antirotationsstift 24 ragt mit seinem Schaft 25 und seiner Spitze 26 in das zu fixierende Knochenfragment 16 hinein und verhindert somit eine Verdrehung des Knochenfragmentes 16 um die Längsachse der Verriegelungsschraube 13. Dadurch, daß der Schaft 25 des Antirotationsstiftes 24 relativ dünn ausgebildet ist und dadurch, daß sich der Antirotationsstift 24 mit der Spitze 26 gegebenenfalls auch tiefer in das Knochenfragment 16 eingraben kann, ist eine Relativverschiebung des Knochenfragmentes 16 zum Antirotationsstift 24 in axialer Richtung möglich, so daß eine gegebenenfalls beim Heilungsprozeß auftretende Annäherung ohne weiteres möglich ist.

## Patentansprüche

1. Vorrichtung zur Versorgung von Knochenbrüchen im gelenknahen proximalen Teil des Femurknochens mit einem in den Markraum des Femurknochens einsetzbaren Verriegelungsnagel, der in seinem oberen Bereich eine quer zu seiner Längsachse verlaufende Durchgangsbohrung aufweist, mit einer in dieser Durchgangsbohrung in axialer Richtung festgelegten Hülse, und mit einer mit einem Schaft in die Hülse eintauchenden und in der Hülse axial verschieblich aufgenommenen Schenkelhalsschraube, wobei die Durchgangsbohrung (7) eine glatte zylindrische Innenwand (9) aufweist, an der die Hülse (17) mit ihrem ebenfalls glatten Außenmantel (19) dicht anliegt, **dadurch gekennzeichnet, daß** die Hülse an einem Ende ein über ihren Außenmantel überstehendes Außengewinde trägt und daß der Durchgangsbohrung (7) in dem Verriegelungsnagel (1) eine stufenförmige Erweiterung (10) vorgelagert ist, die an ihrer Seitenwand (11) mindestens eine im wesentlichen in Umfangsrichtung verlaufende Rippe (12) aufweist, die beim Einschrauben der Hülse (17) in die Durchgangsbohrung (7) zwischen die Gewindegänge des Außengewindes (18) der Hülse (17) eingreift.

2. Vorrichtung nach Anspruch 1, **dadurch gekennzeichnet, daß** die Rippe (12) durch einen Gewindegang eines Innengewindes der Seitenwand (11) gebildet wird.

3. Vorrichtung nach Anspruch 1, **dadurch gekennzeichnet, daß** die Rippe (12) in einer Ebene verläuft, die senkrecht auf der Längsachse der Durchgangsbohrung (7) steht.

4. Vorrichtung nach einem der voranstehenden Ansprüche, **dadurch gekennzeichnet, daß** die Seitenwand (11) der Erweiterung (10) mehrere parallel zueinander verlaufende und nebeneinander liegende Rippen (12) trägt, deren Abstand voneinander gleich ist.

5. Vorrichtung nach einem der voranstehenden Ansprüche, **dadurch gekennzeichnet, daß** sich die Erweiterung (10) nur über einen Teil des Umfanges der Durchgangsbohrung (7) erstreckt.

6. Vorrichtung nach Anspruch 5, **dadurch gekennzeichnet, daß** die Erweiterung (10) bei einer schräg durch den Verriegelungsnagel (1) führenden Durchgangsbohrung (7) an der Seite der Durchgangsbohrung (7) anschließt, die am weitesten über eine senkrecht auf der Längsachse der Durchgangsbohrung (7) stehenden Ebene hervorsteht.

7. Vorrichtung nach einem der voranstehenden Ansprüche, **dadurch gekennzeichnet, daß** parallel zur Durchgangsbohrung (7) eine zweite Durchgangsbohrung (8) in dem Verriegelungsnagel (1) angeordnet ist, welche einen Antirotationsstift (24) aufnimmt.

8. Vorrichtung nach Anspruch 7, **dadurch gekennzeichnet, daß** der Antirotationsstift (24) in die zweite Durchgangsbohrung (8) eingeschraubt ist.

9. Vorrichtung nach Anspruch 7 oder 8, **dadurch gekennzeichnet, daß** der Antirotationsstift (24) über seine aus dem Verriegelungsnagel (1) hervorstehende Länge eine glatte zylindrische Außenwand (25) aufweist.

10. Vorrichtung nach einem der Ansprüche 7 bis 9, **dadurch gekennzeichnet, daß** der Antirotationsstift (24) ein spitzes Ende (26) aufweist.

11. Vorrichtung nach einem der Ansprüche 7 bis 10, **dadurch gekennzeichnet, daß** der Antirotationsstift (24) einen Außendurchmesser (25) aufweist, der wesentlich kleiner ist als der der Hülse (17).

## Claims

1. A device for treating bone fractures in the proximal part of the femur near the joint, having an interlocking nail insertable into the medullary cavity of the femur, which pin has, in its upper region, a through-bore extending obliquely to its longitudinal axis, and having a sleeve fixed in the axial direction within the said through-bore and a femoral-neck screw accommodated so as to penetrate with a shaft into the sleeve and so as to be axially displaceable within the sleeve, the through-bore (7) having a smooth, cylindrical inner wall (9) against which the sleeve (17) fits tightly with its similarly smooth outer surface (19), **characterised in that** the sleeve bears, at one end, an external thread projecting beyond its outer surface and **in that** a stepped expanded portion (10) is positioned in front of the through-bore (7) within the interlocking nail (1), which expanded portion has, on its side wall (11), at least one rib (12) extending substantially in a circumferential direction which, when the sleeve (17) is screwed into the through-bore (7), engages between the turns of the external thread (18) of the sleeve (17).

2. A device according to Claim 1, **characterised in that** the rib (12) is formed by a single turn of an internal thread of the side wall (11).

3. A device according to Claim 1, **characterised in that** the rib (12) extends in a plane perpendicular to the longitudinal axis of the through-bore (7).

4. A device according to any one of the preceding claims, **characterised in that** the side wall (11) of the expanded portion (10) bears a plurality of equally-spaced ribs (12) positioned adjacent to one another and extending parallel to one another.

5. A device according to any one of the preceding claims, **characterised in that** the expanded portion (10) extends only over part of the circumference of the through-bore (7).

6. A device according to Claim 5, **characterised in that** the expanded portion (10) associated with a through-bore (7) passing obliquely through the interlocking nail (1) is contiguous with the side of the through-bore (7) which projects furthest over a plane which is perpendicular to the longitudinal axis of the through-bore (7).

7. A device according to one of the preceding claims, **characterised in that** a second through-bore (8) parallel to the through-bore (7) and which accommodates a counter-rotation pin (24) is arranged within the interlocking nail (1).

8. A device according to Claim 7, **characterised in that** the counter-rotation pin (24) is screwed into the second through-bore (8).

9. A device according to Claim 7 or 8, **characterised in that** the counter-rotation pin (24) has a smooth-cylindrical outer wall (25) over its length projecting out of the interlocking nail (1).

10. A device according to one of Claims 7 or 9, **characterised in that** the counter-rotation pin (24) has a pointed end (26).

11. A device according to one of Claims 7 to 10, **characterised in that** the counter-rotation pin (24) has an external diameter (25) which is substantially smaller than that of the sleeve (17).

## Revendications

1. Dispositif pour traiter des fractures d'os dans la partie proximale, proche de l'articulation, de l'os du fémur comportant un clou de verrouillage pouvant être inséré dans l'espace médullaire de l'os du fémur et qui possède, dans sa partie supérieure, un perçage traversant qui s'étend transversalement par rapport à son axe longitudinal, comportant une douille fixée dans son perçage traversant dans la direction axiale, et une vis du col du fémur, qui pénètre par une tige dans la douille et est logée de manière à être déplaçable axialement dans la douille, et dans lequel le perçage traversant (7) possède une paroi intérieure cylindrique lisse (9), sur laquelle la douille (17) s'applique de façon étanche par son enveloppe extérieure (19) également lisse, **caractérisé en ce que** la douille porte, au niveau d'une extrémité, un filetage extérieur qui fait saillie sur son enveloppe extérieure et qu'en avant du perçage traversant (7) dans le clou de verrouillage (1) est présente une partie élargie de forme étagée (10), qui comporte, sur sa paroi latérale (11), au moins une nervure (12) qui s'étend essentiellement dans la direction circonférentielle et qui, lors du vissage de la vis (17) dans le perçage traversant (7), s'engage entre les filets de vis du filetage extérieur (18) de la douille (17).

2. Dispositif selon la revendication 1, **caractérisé en ce que** la nervure (12) est formée par un filet de vis d'un taraudage de la paroi latérale (11).

3. Dispositif selon la revendication 1, **caractérisé en ce que** la nervure (12) s'étend dans un plan qui est perpendiculaire à l'axe longitudinal du perçage traversant (7).

4. Dispositif selon l'une des revendications précédentes, **caractérisé en ce que** la paroi latérale (11) de la partie élargie (10) porte des nervures (10) qui s'étendent au moins parallèlement entre elles et sont disposées côte-à-côte et dont la distance réciproque est la même.

5. Dispositif selon l'une des revendications précédentes, **caractérisé en ce que** la partie élargie (10) s'étend uniquement sur une partie de la périphérie du perçage traversant (7).

6. Dispositif selon la revendication 5, **caractérisé en ce que** la partie élargie (10) se raccorde, dans le cas d'un perçage traversant (7) disposé obliquement dans le clou de verrouillage (1), sur le côté du perçage traversant (7) qui est le plus en saillie par rapport à un plan qui est perpendiculaire au plan longitudinal du perçage traversant (7).

7. Dispositif selon l'une des revendications précédentes, **caractérisé en ce qu'**en parallèle avec le perçage traversant (7) est disposé un second perçage traversant (8) formé dans le clou de verrouillage (1) et qui possède une tige anti-rotation (24).

8. Dispositif selon la revendication 7, **caractérisé en ce que** la tige anti-rotation (24) est vissée dans le second perçage traversant (8).

9. Dispositif selon la revendication 7 ou 8, **caractérisé en ce que** la tige anti-rotation (24) possède sur sa longueur, qui est en saillie par rapport au clou de verrouillage (1), une paroi extérieure cylindrique (25).

10. Dispositif selon l'une des revendications 7 à 9, **caractérisé en ce que** la tige anti-rotation (24) possède une extrémité pointue (26).

11. Dispositif selon l'une des revendications 7 à 10, **caractérisé en ce que** la tige anti-rotation (24) possède un diamètre extérieur (25) qui est nettement inférieur à celui de la douille (17).
